# EUROPEAN PATENT APPLICATION

(11) **EP 4 560 360 A1**
(43) Date of publication of application: **28.05.2025**
(21) Application number: 23842493.1
(22) Date of filing: 17.07.2023
(51) Int. Cl.: G01T 1/24

(54) **DEVICE AND METHOD FOR MONITORING RADON IN THE AIR IN REAL TIME**

(30) Priority: 18.07.2022 ES 202230660
(71) Applicant: Consejo Superior de Investigaciones Científicas (CSIC), 28006 Madrid (ES)
(72) Inventor: LOZANO FANTOBA, Manuel, 08193 Cerdanyola del Vallès (Barcelona) (ES); FLETA CORRAL, María Celeste, 08193 Cerdanyola del Vallès (Barcelona) (ES); HIDALGO VILLENA, Salvador, 08193 Cerdanyola del Vallès (Barcelona) (ES); HERRANZ ÁLVAREZ, Juan Francisco, 08202 Sabadell (Barcelona) (ES); VIZCAINO DE JULIÁN, Álvaro, 08202 Sabadell (Barcelona) (ES); BENLLIURE ANAYA, José Fernando, 15782 Santiago de Compostela (A Coruña) (ES); CORTINA GIL, María Dolores, 15782 Santiago de Compostela (A Coruña) (ES); LLENERA CRISTOBO, Juan José, 15782 Santiago de Compostela (A Coruña) (ES)
(74) Representative: Pons
(86) International application number: PCT/ES2023/070460
(87) International publication number: WO 2024/018104

(57) **Abstract**

The device comprises: a measuring chamber (1), in the form of a hollow cylinder made of a conducting material, through which an air current to be monitored circulates via natural or forced diffusion; a sensor element (2) with three or more planar semiconductor diodes (3) coplanar with the axial axis of the measuring chamber (1), said diodes being arranged along a section of the axial axis in a polygonal shape; a first polarisation source (41) which polarises the measuring chamber (1); a second polarisation source (42) which polarises the anode relative to the cathode; a signal amplifier (5); a discriminator (6); and a processor (7), on which a method with an instantaneous and/or continuous operating mode is performed, providing radon concentration values over measuring periods varying between 5 and 10 mins.

## Description

### OBJECT OF THE INVENTION

The present invention falls within the technical field of environmental monitoring and radiation measurement equipment using semiconductor devices. The invention is a device and method for monitoring radon in the air in real time with an optimised geometry to maximise efficiency with small sensor surfaces and low polarisation voltages.

### BACKGROUND OF THE INVENTION

Radon (Symbol = Rn; atomic number Z=86) is a naturally occurring radioactive gas. It is the heaviest monatomic gas in nature. This gas is colourless, odourless and tasteless, and being a noble gas, it has virtually no chemical activity compared to other elements in the periodic table. Due to its gaseous nature, it easily diffuses through different media, its diffusion coefficient for air being *D* = 1 × 10⁻¹*c m*²/*s* compared to *D =* 1.13 × 10⁻⁵*c m*²/*s* for water, medium in which it is also soluble.

Three naturally occurring isotopes of radon are known: ²²²*Rn*, ²²⁰*Rn* and ²¹⁹*Rn*, which are referred to respectively as radon, thoron and actinon, for historical reasons. All three are radioactive, which means that they are transformed into other elements by emitting ionising radiation in this process. Each of them forms part of a different natural radioactive chain. Thus, ²²²*Rn* is a link of the radioactive chain of ²³⁸*U*, ²²⁰*Rn* of the chain of ²³²*Th* and ²¹⁹*Rn* of the chain of ²³⁵*U*. These isotopes of radon are the only gaseous elements in their respective radioactive chains, which makes them extraordinarily mobile and facilitates their transfer from the earth matrix in which they originate into the air.

The most stable isotope of radon is ²²²*Rn*, with a half-life a little less than four days (*T*_{1/2} = 3.82*days*), and it is the one that is generically referred to as radon. Radon is a direct daughter of ²²⁶*Ra* through α-decay. Its products of decay form a series of radionuclides, with a short half life, which decay in a matter of hours to ²¹⁰*Pb*, which has a half-life of twenty-two years. Due to their short half-lives, radon daughters quickly reach equilibrium with their parent.

Actinon ( ²¹⁹*Rn*), with a short semi-decay period (*T*_{1/*2*} = 3.96*s*), has a limited migration capacity. This, together with the low isotopic abundance of ²³⁵*U* in natural uranium (0.711 %), makes its presence in the atmosphere negligible. Furthermore, ²³²*Th* is more abundant than ²³⁸*U* in the Earth's crust, but the probability that it disintegrates is much lower, which means that the amount of ²²²*Rn* in the ground is similar to that of ²²⁰*Rn*. However, thoron has a half-life of barely 55.6*s*, which limits its mobility. Therefore, in any atmosphere, the dominant isotope of radon is ²²²*Rn*.

Starting from the hypothesis that the initial concentration of all the daughters of a nucleus is zero, the concentration of any nucleus in a decay chain can be determined by a series of equations known as the Bateman Equations, according to which it takes ²²⁶*Ra* only about thirty days to reach secular equilibrium with ²²²*Rn*, which in turn takes only 3.5 hours to reach secular equilibrium with its short-period daughter.

Under normal conditions, ²²²*Rn* is the largest source of natural radiation exposure in humans (50% of total natural radiation received and 43% of the annual dose according to the UN).

The danger of exposure to high concentrations of radon is not attributed to the gas itself, but to its progeny, which are solid α-particle emitting elements that bind to lung tissue, radiating it.

The products of decay of ²²²*Rn* can be divided into two groups: those with a short half-life, which includes ²¹⁸*P*, ²¹⁴*Ph*, ²¹⁴*Bi* and ²¹⁴*Po*, and those with a long half-life, which includes ²¹⁰*Po*, ²¹⁰*Ph* and ²¹⁰*Bi*. Since the element with the longest half-life in the first group has a half-life of 27 minutes, the entire sequence of decays can be completed before the various regeneration mechanisms present in the human body can eliminate them.

Thus, the kinetic energy of the alpha particles emitted in these decays is dissipated within the cellular matter, either by creating ions and free radicals, or by directly breaking down key molecules (such as DNA), which can lead to serious health problems.

Furthermore, the long half-life group contributes little to the exposure to which the lungs are subjected, since the first nuclide, ²¹⁰*Ph*, has a half-life of 22.3 years, so it will have a high probability of being eliminated by the body itself before it decays.

The main exposure to radon is through inhalation in poorly ventilated rooms. Radon and its daughters can also be assimilated via ingestion, e.g. dissolved in water (in spas and even in private buildings, radon concentrations in both media are related to each other).

Until a few years ago, the term radiation protection was restricted to artificial radioactivity resulting from human manipulation of the atomic nucleus. Radiation levels at a particular location referred to levels above the natural background at that location. Today, this concept has changed, and the public health problem caused by the concentration of radon and its daughters, particularly in homes, has raised awareness of what was hitherto considered a negligible background.

For decades, the WHO (World Health Organization) has been warning about the risks of residential exposure to this gas. Radon is identified as the second leading cause of lung cancer after smoking.

Most measurements of indoor concentrations of radon gas are performed with passive sensors (trace detectors, electrets or activated carbon boxes). In these cases, the sensors are exposed for a time interval ranging from two days to one year in the volume to be studied (e.g. a room in a building). After exposure, the sensor is recovered, analysed in a specialised laboratory and the corresponding radon concentrations are obtained. These are integrated measures, useful for assessing the average dose to which the population has been exposed. This process obviously precludes the possibility of executing immediate remedial actions.

The use of continuous radon monitors is much more attractive, as they allow radon concentrations to be obtained in situ, but the price associated with the equipment or their relatively low detection efficiency are some of the reasons for their minor use. Among the most used active sensors, we identify two detection techniques: ionisation chambers and semiconductor sensors.

Active sensors including ionisation chambers collect a signal from the ionisation of a gas (active volume of the detector) formed when ionising radiation (alpha decay of radon and its daughters) passes through the gas. The chambers can operate in diffusion mode or in forced-air passage mode, which increases the response speed by a significant factor.

These devices can be found commercially and provide very valuable results, as they are systems with a high detection efficiency, and have a very high range of detectable radon activities. On the other hand, they are very expensive and require periodic external recalibration. Therefore, they are intended for use in laboratories and it is recommended that they are handled by specialised personnel.

The use of semiconductor sensors for radon detection is much less widespread and is typically used in low-cost, low-efficiency sensors for domestic use. These sensors are also sensitive to alpha radiation from radon and its daughters.

These sensors are planar silicon diodes with a square or circular geometry that are placed at the bottom of a detection chamber. This detection chamber has a high polarisation (around 400V) with respect to the diode to attract the radioactive ions to the surface of the diode, and allow the detection of alpha decay. The chamber may be in the shape of a straight prism or a cylinder.

This geometry is not optimal, as the electric field is not uniform, there are areas of very low field, there is no symmetry in the arrangement of the diode and the attraction of ions and subsequent detection is not the same throughout the volume.

This, together with the small size of these commercial detectors and the limit imposed by their essentially circular or square geometries, results in a low detection efficiency.

In addition, due to the nature of alpha particles (and the typical energies at which they are produced in the case of this radioactive decay chain), they are only sensitive to the detection of particles whose decay occurs at distances of less than 1 cm from the semiconductor material.

It is also possible to find in the literature references to its use in radon detection in soils. These devices use commercial silicon detectors, the geometry of which is fixed by the manufacturer and is therefore not optimised for this application. Furthermore, its cost is not negligible.

### DESCRIPTION OF THE INVENTION

The present invention describes a device and method for monitoring radon and its daughters in the air in real time, comprising a new geometry of silicon sensor and polarised detection chamber, which solves the above-mentioned problems, allows lower polarisation voltages, increases efficiency and does not increase cost.

As explained above, the danger of exposure to high concentrations of radon is not attributed to the gas itself, but to its progeny, which are solid α-particle emitting elements that bind to lung tissue, radiating it.

In order to detect these particles, the device of the invention is presented, which comprises the conventional elements of a continuously measuring semiconductor radon detector with significant improvements in its geometry.

Firstly, the device comprises a measuring chamber in the form of a hollow cylinder, the cylinder being made of a conductive material, through which air to be analysed for radon circulates.

Secondly, the device comprises a sensor element, which in turn comprises planar diodes made of a semiconductor material with an anode and a cathode, the planar diodes being located on the axial axis of the measuring chamber. The axial axis section on which the diodes are arranged is polygonal, and the minimum number of sides is three. Thus, the distance between the sensitive surface of the diodes and the conductive wall of the measuring chamber is approximately constant, equal to the radius of the cylinder.

The measuring chamber is connected to a first polarisation source, which polarises the measuring chamber with a positive voltage relative to the anode of the diodes, such that the positive ions move towards the diodes. Compared to the conventional configuration of the radon detectors of the state of the art, this geometry allows for a much more uniform field and significantly lower polarisation voltage.

In addition, the diodes are connected to a second polarisation source, which polarises the anode relative to the cathode.

The diodes are connected in parallel and their outputs are fed to a signal amplifier, which pulses each time a decay occurs in the vicinity of the diode and the emitted alpha particle hits the diode. This output pulse is fed to a discriminator which, depending on the energy, emits a digital signal indicating that a decay has occurred.

It is possible to combine all the diodes in a single signal amplifier or use different channels, in order to minimise the electrical noise picked up by the amplifier. In the case of multiple channels, the analogue or digital outputs must be summed into a single output.

The signal discriminator can be of a single level, or have different energy levels, such that the type of isotope that has produced the decay can be discriminated, increasing the accuracy of the conversion of decay rate to concentration.

Finally, the device comprises a processor, configured to convert the pulse count emitted by the discriminator into radon concentration by a fast decision method that uses both the alpha decay of the short half-life isotope ²¹⁸Po and the alpha decay of the longer half-life isotope ²¹⁴Po.

The method, performed in the processor to determine the radon concentration, is based on both the separation by the discriminator of alpha particles emitted by radon and thoron daughters and the identification of the two alpha particles emitted by radon daughters (Polonium 218 and Polonium 214).

Therefore, in a first step of the method, the signals generated in the discriminator are collected, separating those generated by the alpha particles emitted by thoron daughters and those emitted by radon daughters (Polonium 218 and Polonium 214).

Once the data have been obtained, the method comprises two alternative second steps. In a first alternative, an instantaneous operating mode is used, which consists of measuring the time variation of the pulse count generated by the alpha particles emitted by Polonium 218 to determine the concentration of Radon in the air.

In a second alternative, a continuous operating mode is used, in which a secular equilibrium between ²²²Rn, ²¹⁸Po and ²¹⁴Po is reached, and then the Radon concentration in the air is determined as a function of the pulses generated by these particles.

In both the instantaneous operating mode and the continuous operating mode, the processing of the signals of interest and pulse count can be performed over a dynamically adjustable period of time.

Additionally, the device may comprise a forced ventilation module, linked to the measuring chamber, which maximises the amount of air sampled per time unit, increasing the response time to rapid variations in radon gas concentration.

### DESCRIPTION OF THE DRAWINGS

To complement the description that is being made and for the purpose of helping to better understand the features of the invention according to a preferred practical exemplary embodiment thereof, a set of drawings is attached as an integral part of said description in which the following is depicted in an illustrative and non-limiting manner:
Figure 1 shows a cross section of the measuring chamber with the diodes of the sensor element configured in different geometries (triangular, square and hexagonal).
Figure 2 shows the internal structure of a planar semiconductor diode, on the axial axis surrounded by the measuring chamber.
Figure 3 shows a cross section of the measuring chamber with the forced ventilation module.
Figure 4 shows a graph with the energy of the alpha particles generated in the decays of Polonium 218 and Polonium 214.
Figure 5 shows a cross section of the measuring chamber with the field concentrators.

### PREFERRED EMBODIMENT OF THE INVENTION

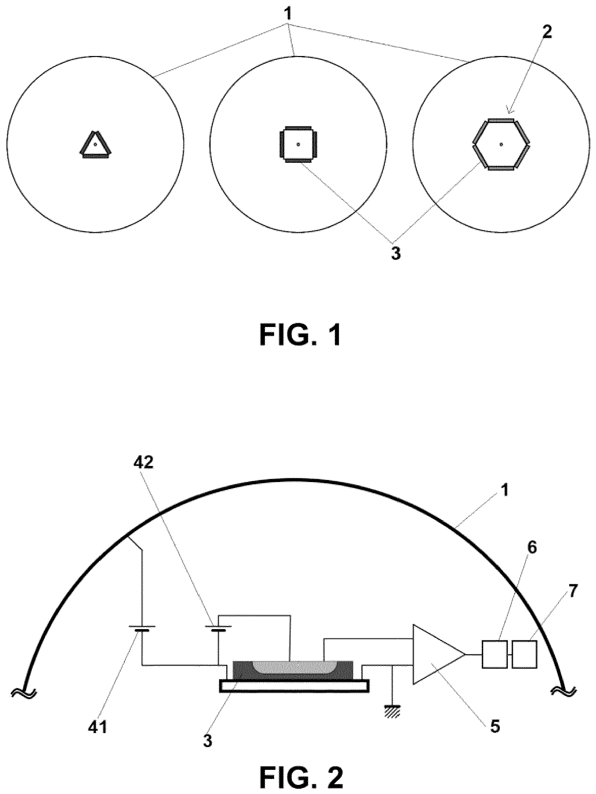

A preferred embodiment of the device and method for monitoring radon in the air in real time, subject matter of the present invention, is described below with the aid of Figures 1 to 5.

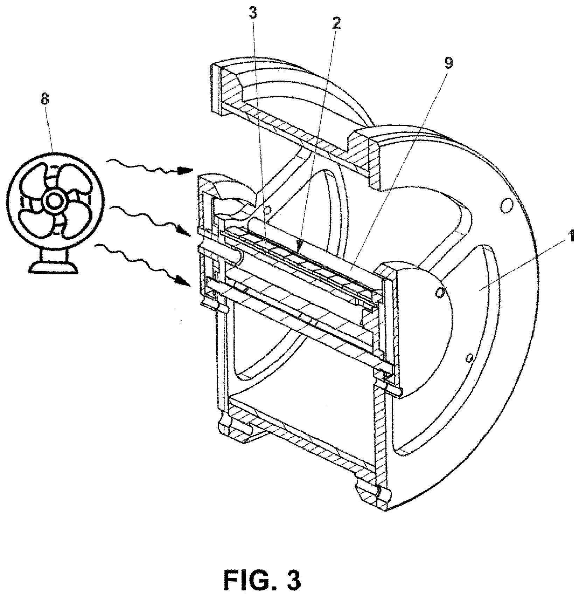

The device, shown in detail in Figure 3, comprises a measuring chamber (1), in the form of a hollow cylinder made of a conducting material, such that the air to be analysed for radon circulates through a space inside the measuring chamber (1). In one embodiment of the invention, the measuring chamber (1) is a metallic aluminium cylinder with a diameter of 8 cm and a length of 10 cm.

Arranged in the inner hollow of the measuring chamber (1), the device comprises a sensor element (2), which in turn comprises planar semiconductor diodes (3). As shown in the cross section of figure 1, the diodes (3) are coplanar with the axial axis of the measuring chamber (1). Specifically, the diodes (3) are positioned on a polygonal axial axis section with a minimum of three sides. Figure 1 shows three possible embodiments of said section: triangular, square and hexagonal.

Preferably, the diodes (3) of the sensor element (2) are diodes made of silicon by a conventional process, which comprises the steps of:
- arranging an N-type silicon wafer,
- oxidising the surface,
- opening openings on the upper face by photolithography and removal of oxide from the rear face,
- implanting P-type impurities on the upper face, through the opening and N-type impurities on the rear face,
- metallising the upper and lower faces,
- photolithographing the metal on the upper face,
- depositing a passivation layer on the upper face,
- opening windows in the passivation layer, and
- cutting the individual diodes (3).

In one aspect of the invention, the sensor element (2) comprises "P over N" type PiN diodes, with a square design, an active area of 0.25*cm*² (surrounded by an extraction ring) and a metal layer completely covering the anode diffusion, with a very shallow P-type detection layer and low reverse leakage current.

The size is chosen so that the diodes (3) are large enough to have acceptable individual sensitivity, but at the same time can be part of a modular structure with design flexibility to increase that sensitivity.

High resistivity n-type silicon wafers with a thickness of 285 micrometres are used to manufacture the diodes (3). The technological process consists of 47 manufacturing steps and 4 photolithographic levels.

The optimal way of distributing these diodes (3) is on three linear plates joined to form a triangular prism structure, as shown in the first embodiment (left) of Figure 1, surrounded by the measuring chamber (1). The diodes (3) are connected in parallel such that each diode can be disconnected individually in the event of a malfunction without affecting the functionality of the others.

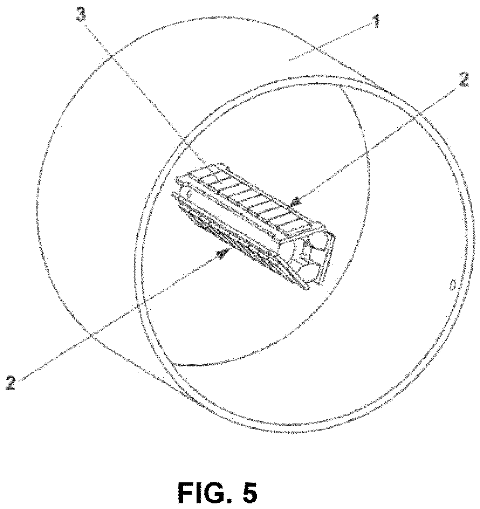

The area occupied by the diodes (3) should ideally be cylindrical, but in practice is approximated by a prism with a triangular cross-section. In this arrangement there are areas at the vertices that are not sensitive to ions. To minimise the number of ions reaching these "blind" areas, the device incorporates field concentrators (9), which may be metal rods, as shown in Figure 5.

As shown in detail in figure 2, a first 200V polarisation source (41) is connected between the measuring chamber (1) and the anode of the diodes (3), which polarises the measuring chamber (1) with a positive voltage relative to the anode of the diodes (3), such that the positive ions of the air current to be analysed move towards the latter. In addition, a second 20 V polarisation source (42) is connected between the anode and cathode of the diodes (3), which polarises the anode relative to the cathode.

The diodes (3) are connected in parallel and their outputs (anode and cathode) are fed to a signal amplifier (5), shown in Figure 2. When alpha particles generated by radon daughters in the vicinity of the diodes (3) impact the same, pulses are emitted and passed to the signal amplifier (5). This generates an amplified output pulse, consisting of a Gaussian signal of 2 µs of time for each detected alpha particle. The area of the Gaussian depends on the energy of the detected particle.

In summary, the electrical output of the diodes (3) takes the form of small current pulses, the intensity of which is proportional to the energy deposited by the particle inside the active volume of the diode (3).

This amplified signal is fed to a voltage discriminator (6) which, depending on the energy, outputs a digital signal indicating that a decay has occurred. The discriminator (6) allows only the spectrum beads with the energies of interest corresponding to the alpha decays of Polonium 218 and Polonium 214 to be selected and no other contributions such as electronic noise.

The signal amplifier (5) and the discriminator (6) therefore convert the current signals to voltage, amplify them, and condition them to be electrically compatible with the operating levels of a pulse counter. The output signal of the discriminator (6) shall be an analogue signal equivalent to the energy integration of the area of the alpha spectrum of interest. This signal shall be converted to radon gas activity units.

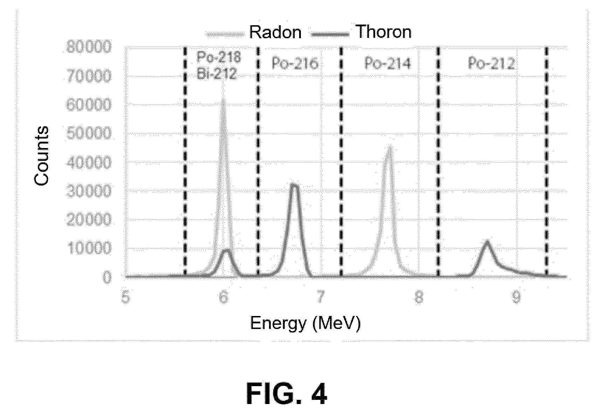

Figure 4 shows the energy spectrum of the alpha particles emitted by the progeny of the main radon isotopes present in the air (Rn 222 - radon, Rn 220 - thoron). The discriminator (6) allows the identification of the different current pulses sent by the diodes (3) associated with each of the alpha decays shown in the figure. This discrimination allows the identification of possible interferences as well as the optimisation of the method for determining the radon concentration detailed below.

Each signal detected within the limits of interest generates a pulse of sufficient duration to be sampled by a processor (7) comprising a pulse counter, connected to the discriminator (6), the sampling times of which are a few milliseconds. The processor (7), in addition to pulse count, also controls the polarisation source (4) and is configured to convert the pulse count emitted by the discriminator (6) into radon concentration.

The method, performed in the processor (7) to determine the radon concentration, is based on both the separation by the discriminator (6) of alpha particles emitted by radon and thoron daughters and the identification of the two alpha particles emitted by radon daughters (Polonium 218 and Polonium 214).

Therefore, in a first step of the method, the signals generated in the discriminator (6) are collected, separating those generated by the alpha particles emitted by thoron daughters and those emitted by radon daughters (Polonium 218 and Polonium 214).

Once the data have been obtained, the method comprises two alternative second steps. In a first alternative, an instantaneous operating mode is used, based on the time variation of the pulse count generated by the alpha particles emitted by Polonium 218 with a half-life of 3.1 min in a short measurement time (less than 10 min) and without the need to reach secular equilibrium with ²²²Rn (27 min).

In a second alternative, a continuous operating mode is used, where after reaching the secular equilibrium between ²²²Rn, ²¹⁸Po and ²¹⁴Po (3.5 h), a more accurate measurement of the variation of the radon concentration is achieved in a short measurement time (similar to the instantaneous mode).

In both the instantaneous operating mode and the continuous operating mode, the processing of the signals of interest and pulse count can be performed over a dynamically adjustable period of time.

Lastly, the device comprises a forced ventilation module (8), linked to the measuring chamber (1), which maximises the amount of air sampled per unit of time, improving the responsiveness of the detector to rapid changes in Rn-222 concentration, compared to waiting for radon to enter the measuring chamber (1) by diffusion.

## Claims

1. A device for monitoring radon in the air in real time, comprising:
- a measuring chamber (1), in the form of a hollow cylinder of a conducting material, designed to be filled by natural diffusion and/or a forced current, with air which may contain Radon-222 (parent) and/or Polonium-218 and Polonium-214, the daughters of Radon, the daughters in turn decaying into alpha particles,
- a sensor element (2), further comprising three or more planar semiconductor diodes (3) connected in parallel, the diodes (3) comprising an anode and a cathode, the diodes (3) being coplanar with the axial axis of the measuring chamber (1), and the diodes (3) being arranged along a section of the axial axis in a polygonal shape,
- a first polarisation source (41) connected between the measuring chamber (1) and the anode of the diodes (3), which polarises the measuring chamber (1) with a positive voltage relative to the anodes of the diodes (3),
- a second polarisation source (42), connected between the anode and cathode of the diodes (3), which polarises the anode relative to the cathode,
- a signal amplifier (5) connected to the diodes (3) which generates a pulse of a certain energy each time a decay occurs and an emitted alpha particle impacts the diodes (3), the energy varying depending on which element generated the alpha particle,
- a discriminator (6) connected to the signal amplifier (5) which, depending on the pulse energy, detects which element has generated the alpha particle, generating signals of interest,
- a processor (7), connected to the polarisation sources (41, 42), comprising a pulse counter that generates a pulse count from the signals of interest from the discriminator (6), the processor (7) being configured to convert the pulse count together with the signals of interest into radon concentration data.

2. The device of claim 1, further comprising a forced ventilation module (8), linked to the measuring chamber (1), which maximises the air current sampled per unit of time.

3. The device of claim 1, wherein the diodes (3) are arranged in an axial section of the measuring chamber (1) according to a polygonal shape selected from: triangular, square and hexagonal.

4. The device of claim 1, wherein the diodes (3) are three linear plates joined to form a triangular prism structure.

5. The device of claim 1, wherein the diodes (3) are silicon diodes.

6. The device of claim 1, further comprising field concentrators (9) arranged between the diodes (3).

7. A method for monitoring Radon in the air in real time, using the device of any of the preceding claims, comprising the steps of:
- collecting the signals of interest from the discriminator (6), and collecting the pulse count from the processor (7),
- processing the signals of interest and pulse count:
∘ instantaneously, determining a time variation of the pulse count generated by the alpha particles emitted by Polonium 218 to determine the concentration of Radon in the air, and/or
∘ continuously, waiting until a secular equilibrium between Polonium-218 and Polonium-214 is reached, and combining the pulse count together with the signals of interest to determine the Radon concentration in the air.

8. The method of claim 7, wherein the processing step of the signals of interest and pulse count is performed over a dynamically adjustable period of time.
